# EUROPÄISCHE PATENTSCHRIFT

(11) **EP 0 969 095 B1**
(45) Veröffentlichungstag und Bekanntmachung des Hinweises auf die Patenterteilung: **29.09.2004**
(21) Anmeldenummer: 99111574.2
(22) Anmeldetag: 15.06.1999
(51) Int. Cl.: C12N 15/60, C12N 9/88, C12P 13/00

(54) **(S)-Hydroxynitrillyasen mit verbesserter Substanzakzeptanz und deren Verwendung**
(S)-Hydroxynitrile lyases with improved substrate acceptance and uses thereof
(S)-Hydroxynitrile lyases présentant une acceptation améliorée pour le substrat et leurs utilisations

(30) Priorität: 02.07.1998 AT 115998
(43) Veröffentlichungstag der Anmeldung: 05.01.2000
(73) Patentinhaber: DSM Fine Chemicals Austria Nfg GmbH & Co KG, 4021 Linz (AT)
(72) Erfinder: Effenberger, Franz, 70597 Stuttgart (DE); Lauble, Peter, 10965 Berlin (DE); Bühler, Holger, 71640 Ludwigsburg (DE); Wajant, Harald, 70771 Leinfelden-Echterdingen (DE); Förster, Siegfried, 70569 Stuttgart (DE); Schwab, Helmut, 8043 Graz (AT); Kratky, Christoph, 8010 Graz (AT); Wagner, Ulrike, 8010 Graz (AT); Steiner, Ernst, 8144 Tobelbad (AT)
(74) Vertreter: Lindinger, Ingrid

(56) Entgegenhaltungen:
- WO-A-97/03204
- WAJANT H ET AL: "IDENTIFICATION OF POTENTIAL ACTIVE-SITE RESIDUES IN THE HYDROXYNITRILE LYASE FROM MANIHOT ESCULENTA BY SITE-DIRECTED MUTAGENESIS" JOURNAL OF BIOLOGICAL CHEMISTRY, AMERICAN SOCIETY OF BIOLOGICAL CHEMISTS, BALTIMORE, MD, US, Bd. 271, Nr. 42, 18. Oktober 1996 (1996-10-18), Seiten 25830-25834, XP000995344 ISSN: 0021-9258
- HASSLACHER MEINHARD ET AL: "Hydroxynitrile lyase from Hevea brasiliensis: Molecular characterization and mechanism of enzyme catalysis." PROTEINS STRUCTURE FUNCTION AND GENETICS, Bd. 27, Nr. 3, 1997, Seiten 438-449, XP001083603 ISSN: 0887-3585
- WAGNER U G ET AL: "Mechanism of cyanogenesis: the crystal structure of hydroxynitrile lyase from Hevea brasiliensis." STRUCTURE (LONDON, ENGLAND) ENGLAND 15 JUL 1996, Bd. 4, Nr. 7, 15. Juli 1996 (1996-07-15), Seiten 811-822, XP002203197 ISSN: 0969-2126
- HASSLACHER M ET AL: "MOLECULAR CLONING OF THE FULL-LENGTH CDNA OF (S)-HYDROXYNITRILE LYASE FROM HEVEA BRASILIENSIS. FUNCTIONAL EXPRESSION IN ESCHERICHIA COLI AND SACCHAROMYCES CERECISIAE AND IDENTIFICATION OF AN ACTIVE SITE RESIDUE" JOURNAL OF BIOLOGICAL CHEMISTRY, AMERICAN SOCIETY OF BIOLOGICAL CHEMISTS, BALTIMORE, MD, US, Bd. 271, Nr. 10, 8. März 1996 (1996-03-08), Seiten 5884-5891, XP002022209 ISSN: 0021-9258
- LAUBLE HANSPETER ET AL: "Structure determinants of substrate specificity of hydroxynitrile lyase from Manihot esculenta." PROTEIN SCIENCE, Bd. 11, Nr. 1, Januar 2002 (2002-01), Seiten 65-71, XP001080762 ISSN: 0961-8368

## Beschreibung

Die Durchführung chemischer Reaktionen unter Zuhilfenahme biologischer Katalysatoren erlangt zunehmend Bedeutung, vor allem in solchen Anwendungsbereichen, in denen die bei Enzymen häufig ausgeprägte Eigenschaft, bei Reaktionen mit chiralen oder prochiralen Komponenten bevorzugt eines der beiden Enantiomeren umzusetzen, genutzt werden kann.

Zu dieser Gruppe Enzyme zählt die Familie der Hydroxynitrillyasen (HNL), unter anderem HNL aus *Hevea brasiliensis* (HbHNL) und *Manihot esculenta* (MeHNL). Beide Enzyme zeigen eine hohe Sequenzidentität und gehören zu den Proteinen des "α/β-Hydrolase fold"-Typs, die eine charakteristische Tertiärfaltung und eine sogenannte katalytische Triade mit Asparaginsäure, Serin und Histidin als aktives Zentrum aufweisen. Das aktive Zentrum liegt dabei am inneren Ende eines hydrophoben Kanals (U. Wajner et al., 1996, Structure, 4, 811-822).

HbHNL und MeHNL eignen sich im Prinzip für die Umsetzung einer Vielzahl von Carbonylverbindungen, wie etwa aliphatische, alicyclische, ungesättigte, aromatische, sowie heteroaromatische Aldehyde und Ketone, zu den entsprechenden (S)-Cyanhydrinen. Da HNLs eine immer größere Bedeutung als Biokatalysatoren zur Herstellung von (S)-Cyanhydrinen erlangen, wird ständig versucht, deren katalytische Aktivität und Substratakzeptanz zu verbessern. Bisher erhältliche HNLs weisen insbesondere bei Edukten mit sperrigen Resten eine nicht zufriedenstellende Substratakzeptanz auf, wodurch die entsprechenden Cyanhydrine entweder mit geringer Umsatzgeschwindigkeit und/oder geringem Enantiomerenüberschuß erhalten werden.

Aufgabe der Erfindung war es demnach, (S)-Hydroxynitrillyasen mit einer verbesserten Substratakzeptanz bereitzustellen.

Gegenstand der Erfindung sind demnach (S)-Hydroxynitrillyasen mit verbesserter Substratakzeptanz, welche sich von jenen aus *Hevea brasiliensis* und *Manihot esculenta* ableiten, die dadurch gekennzeichnet sind, dass ein oder mehrere Tryptophanreste innerhalb des zum aktiven Zentrum führenden hydrophoben Kanals durch einen Aminosäurerest aus der Gruppe Alanin, Glycin, Valin oder Phenylalanin substituiert sind.

Die erfindungsgemäßen HNLs sind Mutanten von (S)-HNLs aus *Hevea brasiliensis* (HbHNL) oder *Manihot esculenta (MeHNL)*, die aus gentechnisch modifizierten Mikroorganismen wie *Pichia pastoris*, *Saccharomyces cerevisiae*, oder *Escherichia coli* erhalten werden können. (WO 97/03204)
Die zu modifizierenden rekombinanten HNL's können dabei auch in einer verkürzten Sequenz, die beispielsweise durch Entfernen der ersten Aminosäure(n) in der Sequenz erhalten wird, vorliegen.
Die Mutanten weisen eine veränderte Sequenz derjenigen Aminosäuren auf, die den zum aktiven Zentrum führenden hydrophoben Kanal bilden.

Dabei sind ein oder mehrere Tryptophanreste durch einen Aminosäurerest aus der Gruppe Alanin, Glycin, Valin oder Phenylalanin substituiert.
Bevorzugt sind Mutanten die in Position 128 (Tryptophan) der unverkürzten Sequenz von HbHNL oder MeHNL durch Alanin oder Phenylalanin substituiert sind.

Die Herstellung der erfindungsgemäßen HNLs erfolgt durch funktionelle Überexpression in gentechnisch modifizierten Mikroorganismen wie *Pichia pastoris*, *Saccharomyces cerevisiae* oder *Escherichia coli*, beispielsweise analog M. Hasslacher et al., J. Biol. Chem. 1996, 271, 5884 oder Wajant, H. und Pfizenmaier, K. 1996, J. Biol. Chem., 25830 - 25834. Die Mutation wird beispielsweise mittels Quick Change Site-Directed Mutagenesis Kit (Stratagene) nach Anleitung des Herstellers durchgeführt. Der Quick Change Site-Directed Mutagenesis Kit ist ein gebrauchsfertiges System zur Herstellung von spezifischen Mutanten und wird beispielsweise von der Fa. Stratagene Cloning Systems, La Jolla, CA (USA) kommerziell vertrieben.

Die Aufreinigung der resultierenden HNLs erfolgt nach Standartmethoden, beispielsweise analog Wajant, H. Pfizenmaier, K. 1996, J. Biol. Chem., 25830-25834.

Die erfindungsgemäßen HNLs eignen sich zur Herstellung von (S)-Cyanhydrinen in gegenüber dem Stand der Technik verbesserter Umsatzgeschwindigkeit und/oder mit höherem Enantiomerenüberschuss.
Insbesondere werden die erfindungsgemäßen HNLs bei aliphatischen und aromatischen Aldehyden und Ketonen als Substrat eingesetzt.
Unter aliphatischen Aldehyden sind dabei bevorzugt gesättigte oder ungesättigte, verzweigte oder cyclische Aldehyde mit 2 bis 20 C-Atomen zu verstehen.

Besonders bevorzugt sind gesättigte oder ungesättigte verzweigte Aldehyde mit 4 bis 18 C-Atomen.
Die aliphatischen und aromatischen Aldehyde können unsubstituiert oder durch unter den Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Nitro- oder Azidogruppen substituiert sein.

Beispiele für geeignete aliphatische Aldehyde sind Hexanal, Hexenal, Heptanal, Propanal, Octanal, Octenal, 2-Methylpropanal.
Geeignete aromatische oder heteroaromatische Substrate sind etwa Benzaldehyd bzw. verschieden substituierte Benzaldehyde, wie etwa 3-Phenoxybenzaldehyd, 4-Fluor-3-phenoxybenzaldehyd, 2-Chlorbenzaldehyd, 2-Nitrobenzaldehyd, 4-Methylbenzaldehyd, u.s.w.

Die Substrate werden in Anwesenheit der erfindungsgemäßen HNLs mit einem Cyanidgruppendonor umgesetzt.

Als Cyanidgruppendonor kommen Blausäure, Alkali-Cyanide oder ein Cyanhydrin der allgemeinen Formel

R₁R₂C(OH)(CN) Formel I

in Betracht. In der Formel I bedeuten R₁ und R₂ unabhängig voneinander Wasserstoff oder eine unsubstituierte Kohlenwasserstoffgruppe, oder R₁ und R₂ gemeinsam eine Alkylgruppe mit 4 oder 5 C-Atomen, wobei R₁ und R₂ nicht gleichzeitig Wasserstoff bedeuten. Die Kohlenwasserstoffgruppen sind aliphatische oder aromatische, bevorzugt aliphatische Gruppen. Bevorzugt bedeuten R₁ und R₂ Alkylgruppen mit 1 - 6 C-Atomen, ganz bevorzugt ist der Cyanidgruppendonor Acetoncyanhydrin.

Die Herstellung des Cyanidgruppendonors kann nach bekannten Verfahren erfolgen. Cyanhydrine, insbesondere Acetoncyanhydrin, sind auch käuflich zu erwerber.
Bevorzugt wird Blausäure (HCN), KCN, NaCN, oder Acetoncyanhydrin, besonders bevorzugt Blausäure als Cyanidgruppendonor eingesetzt.

Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Die Umsetzung kann im organischen, wässrigen oder 2-Phasensystem oder in Emulsion durchgeführt werden.
Als wässriges System wird eine wässrige, die erfindungsgemäße HNL enthaltende Lösung oder Pufferlösung verwendet. Beispiele dafür sind Na-Citratpuffer, Phosphatpuffer u.s.w.

Als organisches Lösungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden Methyl-tert.Butylether (MTBE), Diisopropylether, Dibutylether und Ethylacetat oder deren Gemisch eingesetzt.
Die erfindungsgemäßen HNLs können dabei entweder als solche oder immobilisiert in dem organischen Verdünnungsmittel vorliegen, die Umsetzung kann jedoch auch in einem Zweiphasensystem oder in Emulsion mit nicht-immobilisierter HNL erfolgen.

### Beispiel 1:

Herstellung von in Position 128 mutierter HbHNL
Spezifische Mutanten wurden unter Verwendung des QuikChange™ Site-Directed Mutagenesis Kit (Stragagene Cloning Systems, La Jolla, CA, USA) hergestellt. Neben einem Aminosäureaustausch in der Position 128 des HNL Proteins wurde über eine stille Mutation zusätzlich noch eine Schnittstelle für das Restriktionsenzym AccIII eingeführt.

Dazu wurden folgende Oligonukleotide verwendet:
a) Zur Herstellung der Mutante W128A: 
W128AFOR: 5' GCTCATGGAGGTGTTTCCGGACGCAAAGACACCACG 3'
W128AREV: 5' CGTGGTGTCTTTTGCGTCCGGAAACACCTCCCATGAGC 3'
b) Zur Herstellung der Mutante W128F:

W128FFOR: 5' GCTCATGGAGGTGTTTCCGGACTTCAAAGACACCACG 3'
W128FREV: 5' CGTGGTGTCTTTGAAGTCCGGAAACACCTCCCATGAGC 3'

Als Template für die Mutagenese-Reaktion wurde das rekombinante Plasmid pHNL104, das die cDNA des *hnl*-Gens von *Hevea brasiliensis* enthält (Plasmidherstellung analog Hasslacher et al. 1996 J.Biol.Chem. 271, 5884) verwendet. Das mutierte Plasmid wurde anschließend in *Escherichia coli* (Epicurian Coli® XL1-Blue supercompetent cells, Stratagene Cloning Systems, La Jolla, CA, USA) transformiert.

Aus mehreren Transformanten wurde dann die Plasmid-DNA isoliert und diese mit dem Restriktionsenzym AccIII auf das Vorhandensein der mit der Mutation eingeführten AccIII-Schnittstelle überprüft. Positive Klone wurden dann im Bereich der *hnl-* cDNA einer Sequenzanalyse unterzogen, um das Vorhandensein der gewünschten Mutation an der Position 128 zu verifizieren und um ausschließen zu können, daß in anderen Bereichen des *hnl*-Gens unerwünschte Mutationen eingeführt wurden.
Aus dem entsprechenden Plasmid wurde dann durch Verdau mit der Restriktionsendonuclease EcoRI und nachfolgender Agarosegelelektrophorese ein Fragment, das die jeweilige mutierte HNL kodiert, gewonnen. Dieses Fragment wurde in die mit EcoRI linearisierte und mit alkalischer Phosphatase dephosphorylierte DNA des Expressionsvektors pHIL-D2 (Invitrogen Corporation, Carlsbad, CA, USA) ligiert und die rekombinante DNA in *Escherichia coli* SURE@ (Stratagene Cloning Systems, La Jolla, CA, USA) transformiert.
Wiederum wurde aus mehreren Transformanten die Plasmid-DNA präpariert und das Vorhandensein bzw. die Orientierung der *hnl* cDNA bezüglich des *aox*I - Promotors von pHIL-D2 mit Hilfe der Restriktionsendonuklease NdeI überprüft. Plasmid-DNA aus geeigneten Klonen wurde dann mit dem Restriktionsenzym NotI linearisiert und in weiterer Folge mittels Elektrotransformation in den *Pichia pastoris* Stamm GS115 eingebracht, wobei auf die Komplementation der Histidindes verwendeten Wirtsstammes selektioniert wurde. So erhaltene Transformanten wurden anschließend auf verringertes Wachstum auf Minimal Methanol Medium geprüft. Mehrere solcher Mut^{s}-Transformanten wurden dann hinsichtlich der Fähigkeit zur Expression von HNL-Protein getestet, und ein geeigneter Expressionsstamm wurde für jede der beiden HNL-Mutanten ausgewählt. Diese Arbeiten wurden analog den im Manual zum Pichia Expression Kit (Invitrogen Corporation, Carlsbad, CA, USA) detailliert beschriebenen Experimenten durchgeführt.

Die Integration der *hnl* cDNA ins *Pichia pastoris* Genom und das Vorhandensein der spezifischen Mutation in den beiden Expressionsstämmen wurde durch Sequenzierung eines mit PCR amplifizierten DNA Fragmentes überprüft. Als Template für die PCR diente chromosomale DNA, die nach der bei Ausubel et. al., Current Protocols in Molecular Biology, Vols. 1-3, Greene Publishing Associates and Wiley-Interscience, New York, 1995, beschriebenen Methode isoliert wurde. Die Sequenzen der PCR-Primer waren wie folgt:

PP5AOX1 5' GACTGGTTCCAATTGACAAGC 3'
PP3AOX1 5' GCAAATGGCATTCTGACATCC 3'

Die Fermentation der Expressionsstämme, sowie die Isolierung und Reinigung der mutierten HNL-Proteine erfolgte wie bei Hasslacher et al. 1997, Protein Expression and Purification, 11, 61-71, beschrieben.

### Beispiel 2

### Herstellung von in Position 128 mutierter MeHNL.

Die Mutation wurde in pQE4-MeHNLwt mittels Quick-Change Site-Directed Mutagenesis Kit (Stratagene Cloning Systems, La Jolla, CA, USA) transformiert. Dazu wurden 2 komplementäre Primer, entsprechend den Nukleotiden 383-435 von MeHNL (5'AAG CTT TTG GAG TCG TTT CCT GAC GCG AGA GAC ACA GAG TAT TTT ACG TTC AC 3') die die gewünschte Mutation (unterstrichen) enthalten mittels Pfu DNA polymerase verlängert und das so erhaltene Produkt mit Dpnl behandelt. Die mutierten Plasmide wurden anschließend in E.coli XL1-Blue transformiert. Die Mutanten wurden durch Sequenzanalyse mittels einer modifizierten Bestimmungsmethode analog Sanger et al. (1977) Proc. Natl. Acad. Sci. U.S.A.,74, 5463-5467 mit T7DNA analysis system (Pharmacia) kontrolliert.

### Beispiel 3

### Herstellung von (S)-3-Phenoxybenzaldehydcyanhydrin

(S)-3-Phenoxybenzaldehydcyanhydrin (PBAC) wurde durch Umsetzung von 10 bzw. 5 mmol m-Phenoxybenzaldehyd (PBA), und 14,4 bzw. 7,7 mmol HCN in tert. Butylmethylether (MTBE) in Anwesenheit von in Position 128 durch Alanin substituierte MeHNL W128A bzw. von rekombinanter MeHNL (J.Hughes et al., Arch.Biochem.Biophys.1994, 311 (2), pp. 496-502) oder von rekombinanter HbHNL - Bsp. Mutant erhalten.
Dazu wurde jeweils 1,3 ml Enzymlösung (0,26 ml für HbHNL) mit 8,3 ml 50 mMol Na-Citratpuffer 5,4 verdünnt (bzw. 9,74 ml, dest. H₂O für HbHNL), die entsprechende Menge PBA (1,98 g/1 g) und 3 bzw. 1,5 ml MTBE zugegeben und anschließend 0,06 ml/ml Aldehyd HCN rasch zugetropft. Der Reaktionsverlauf wurde mittels IP-Kontrolle über die Abnahme des Aldehydgehaltes verfolgt. Nach 2 Stunden wurden die Reaktionen abgebrochen. Zur Aufarbeitung wurden die Reaktionslösungen mit jeweils 2,5 ml MTBE verdünnt, geschüttelt und zentrifugiert.

Die Ergebnisse sind aus Tabelle 1 ersichtlich.

**Tabelle 1**

| HNL | IU/ml | mmol PBA | Gehalt PBAC [%] | ee [%] |
|---|---|---|---|---|
| MeHNL (Wildtyp) | 1092 | 10 | 82 | n.e. |
| MeHNL (Wildtyp) | 1092 | 5 | 88,5 | 97,03 |
| MeHNL (W128A) | 315 | 10 | 98,7 | n.e. |
| MeHNL (W128A) | 315 | 5 | 97 *) | 97 |
| HbHNL | 5200 | 10 | 83 | n.e. |
| HbHNL | 5200 | 5 | 84,5 | n.e. |

| | | | | |
|---|---|---|---|---|
| *) nach 1,5 h | | | | |
| n.e.) nicht ermittelt | | | | |

### Beispiel 4

Vergleich rekombinante MeHNL (Wildtyp) aus Manihot esculenta und mutante MeHNL W128A im organischen Medium.
Analog Bsp. 3 wurden 3 mg Enzym, 100 mg Nitrocellulose (vorbehandelt mit 20 mMol Citratpuffer pH 3.3) 1 mmol Substrat und 150 µl HCN in 5 ml Diisopropylether umgesetzt.

Die Ergebnisse sind aus Tabelle 2 ersichtlich

**Tabelle 2**

| | MeHNL | | | W128A | | |
|---|---|---|---|---|---|---|
| Substrat | t [h] | Ausbeute [%] | ee [%] | t [h] | Ausbeute [%] | ee [%] |
| Nonanal | 6 | 35 | 81,1 | 6 | 87,3 | 80,2 |
| Heptanal | 1 | 99 | 80 | 1 | 99,5 | 88 |
| 2-Methylbutanal | 4,5 | 89 | 93,6 | 4,5 | 99 | 96,6 |
| 2-Hexenal | 3,2 | 21,1 | >99 | 3,2 | 85 | 96 |
| n-PBA | 7 | 30 | 99 | 8 | 82 | 99 |
| p-HBA | 2 | 31 | >99 | 2 | 60 | >99 |
| CPA | 1 | 90,7 | 99 | 1 | 94,3 | 99 |
| PPA | 0,3 | 79 | 67 | 0,3 | 93 | 86 |
| Phenylaceton | 4 | 69,8 | 99 | 4 | 81,6 | 96 |
| BMK | 4,5 | 100 | 52 | 4,5 | 100 | 78 |
| BEK | 5 | 40 | 45 | 5 | 46 | 82 |
| p-HBA p-Hydroxybenzaldehyd PPA Phenylpropionaldehyd CPA Cyclopent-1-enylacetaldehyd BMK Butylmethylketon BEK Butylethylketon | | | | | | |

### Beispiel 5

Vergleich rekombinante MeHNL und mutante MeHNL W128A im wäßrigen Puffersystem
Analog Bsp. 3 wurden 1,2 mg Enzym mit 0,5 mmol Substrat in 2,5 ml eines 0,5 M Na-Citratpuffers pH 3.8 mit 1 mmol KCN in 3,5 ml 0,5 M Na-Citratpuffer pH 3.8 umgesetzt.
Die Ergebnisse sind aus Tabelle 3 ersichtlich

**Tabelle 3**

| | MeHNL | | | W128A | | |
|---|---|---|---|---|---|---|
| Substrat | t [h] | Ausbeute [%] | ee [%] | t [h] | Ausbeute [%] | ee [%] |
| FPBA | 4 | 11 | 99 | 4 | 47 | 95 |
| PBA | 22 | 66 | 97 | 22 | 75 | 92 |
| m-HBA | 3 | 74 | 0 | 3 | 80 | 0 |
| p-HBA | 1 | 76 | 92 | 1 | 71 | 96 |
| PPA | 1.2 | 60 | 21 | 1.2 | 94 | 90 |
| FPBA 4-Fluor-3-Phenoxybenzaldehyd | | | | | | |

## Patentansprüche

1. (S)-Hydroxynitrillyasen mit verbesserter-Substratakzeptanz, welche sich von jenen *aus Hevea brasiliensis* und *Manihot esculenta* ableiten, **dadurch gekennzeichnet, dass** ein oder mehrere Tryptophanreste innerhalb des zum aktiven Zentrum führenden hydrophoben Kanals durch einen Aminosäurerest aus der Gruppe Alanin, Glycin, Valin oder Phenylalanin substituiert sind.

2. (S)-Hydroxynitrillyasen nach Anspruch 1, **dadurch gekennzeichnet, dass** sie sich von (S)-Hydroxynitrillyasen aus *Hevea brasiliensis* und *Manihot esculenta* ableiten, die in vollständiger oder in verkürzter Sequenz vortiegen.

3. (S)-Hydroxynitrillyasen nach Anspruch 1, **dadurch gekennzeichnet, dass** ein oder mehrere Tryptophanreste innerhalb des zum aktiven Zentrum führenden hydrophoben Kanals durch Alanin oder Phenylalanin substituiert sind.

4. (S)-Hydroxynitrillyasen nach Anspruch 1 bis 3, **dadurch gekennzeichnet, dass** in Position 128 der unverkürzten Sequenz der (S)-Hydroxynitrillyasen der Tryptophanrest durch Alanin oder Phenylalanin substituiert ist.

5. Verfahren zur Herstellung von (S)-Hydroxynitrillyasen nach Anspruch 1, **dadurch gekennzeichnet, dass** innerhalb des zum aktiven Zentrum führenden hydrophoben Kanals eine Mutation mittels Quick Change Site-Directed Mutagenesis durchgeführt wird.

6. Verwendung von (S)-Hydroxynitrillyasen nach Anspruch 1, zur Herstellung von (S)-Cyanhydrinen.

7. Verfahren zur Herstellung von (S)-Cyanhydrinen, **dadurch gekennzeichnet, dass** aliphatische und aromatische Aldehyde und Ketone in Anwesenheit eines Cyanidgruppendonors mit (S)-Hydroxynitrillyasen nach Anspruch 1 umgesetzt werden.

8. Verfahren nach Anspruch 7, **dadurch gekennzeichnet, dass** die Umsetzung in einem organischen, wässrigen oder in einem 2-Phasensystem oder in Emulsion durchgeführt wird.

## Claims

1. (S)-Hydroxynitrile lyases which have improved substrate acceptance and which are derived from the *Hevea brasiliensis* or *Manihot esculenta* (S)-hydroxynitrile lyases, **characterized in that** one or more tryptophan residues within the hydrophobic channel leading to the active centre have been replaced with an amino acid residue from the group consisting of alanine, glycine, valine or phenylalanine.

2. (S)-Hydroxynitrile lyases according to Claim 1, **characterized in that** they are derived from (S)-hydroxynitrile lyases which are obtained from *Hevea brasiliensis* or *Manihot esculenta* and are full-length or truncated.

3. (S)-Hydroxynitrile lyases according to Claim 1, **characterized in that** one or more tryptophan with residues in the hydrophobic channel leading to the active centre have been replaced with alanine or phenylalanine.

4. (S)-Hydroxynitrile lyases according to Claims 1 to 3, **characterized in that** the tryptophan residue in position 128 of the full-length sequence of the (S)-hydroxynitrile lyases has been replaced with alanine or phenylalanine.

5. Process for preparing (S)-hydroxynitrile lyases according to Claim 1, **characterized in that** Quick Change Site-Directed Mutagenesis is used to carry out a mutation within the hydrophobic channel leading to the active centre.

6. Use of (S)-hydroxynitrile lyases according to Claim 1 for preparing (S)-cyanohydrins.

7. Process for preparing (S)-cyanohydrins, **characterized in that** aliphatic and aromatic aldehydes and ketones are reacted, in the presence of a cyanide group donor, with (S)-hydroxynitrile lyases according to Claim 1.

8. Process according to Claim 7, **characterized in that** the reaction is carried out in an organic, aqueous or 2-phase system or in an emulsion.

## Revendications

1. (S)-Hydroxynitrile lyases présentant une acceptation améliorée pour le substrat, qui dérivent de celles de *Hevea brasiliensis* et de *Manihot esculenta*, qui sont **caractérisées en ce qu'**un ou plusieurs résidus tryptophane dans le canal hydrophobe conduisant au centre actif, sont remplacés par un résidu acide aminé du groupe de l'alanine, de la glycine, de la valine ou de la phénylalanine.

2. (S)-Hydroxynitrile lyases selon la revendication 1, **caractérisées en ce qu'**elles dérivent de (S)-hydroxynitrile lyases de *Hevea brasiliensis* et de *Manihot esculenta,* qui se présentent avec une séquence complète ou raccourcie.

3. (S)-Hydroxynitrile lyases selon la revendication 1, **caractérisées en ce qu'**un ou plusieurs résidus tryptophane dans le canal hydrophobe conduisant au centre actif, sont remplacés par un résidu acide aminé du groupe de l'alanine ou de la phénylalanine.

4. (S)-Hydroxynitrile lyases selon les revendications 1 à 3, **caractérisées en ce qu'**en la position 128 de la séquence non raccourcie des (S)-hydroxynitrile lyases, le résidu tryptophane est substitué par alanine ou phénylalanine.

5. Procédé de préparation de (S)-hydroxynitrile lyases selon la revendication 1, **caractérisé en ce que** l'on réalise dans le canal hydrophobe conduisant au centre actif, une mutation à l'aide du Quick Change Site-Directed Mutagenesis.

6. Utilisation de (S)-hydroxynitrile lyases selon la revendication 1, pour la préparation de (S)-cyanhydrines.

7. Procédé de préparation de (S)-cyanhydrines, **caractérisé en ce que** l'on fait réagir des aldéhydes et des cétones aliphatiques et aromatiques en présence d'un donneur de radical cyanure avec les (S)-hydroxynitrile lyases selon la revendication 1.

8. Procédé selon la revendication 7, **caractérisé en ce que** la réaction est réalisée dans un système organique, aqueux ou biphasique ou en émulsion.
